# EUROPEAN PATENT SPECIFICATION

(11) **EP 4 409 257 B1**
(45) Date of publication and mention of the grant of the patent: **10.06.2026**
(21) Application number: 22786373.5
(22) Date of filing: 28.09.2022
(51) Int. Cl.: G01N 1/14, G01N 1/18, B01L 3/00, C12M 1/32, G01N 1/00, G01N 21/77, G01N 35/00, G01N 1/10, G01N 21/552

(54) **ON-LINE BIOMOLECULAR ANALYSIS FOR MONITORING OR CONTROLLING BIOPROCESSES**
BIOMOLEKULARE ONLINE-INTERAKTIONSANALYSE ZUR ÜBERWACHUNG ODER STEUERUNG VON BIOPROZESSEN
ANALYSE EN LIGNE D'INTERACTIONS BIOMOLÉCULAIRES POUR LA SURVEILLANCE OU LE CONTRÔLE DES BIOPROCÉDÉS

(30) Priority: 28.09.2021 EP 21199383
(43) Date of publication of application: 07.08.2024
(73) Proprietor: Sartorius Stedim Biotech GmbH, 37079 Göttingen (DE)
(72) Inventor: SCHOLZ, Jochen, 37077 Göttingen (DE); GRIMM, Christian, 37308 Heilbad Heiligenstadt (DE); DUROUS, Laurent, 51373 Leverkusen (DE); PIEL, Mike Michael, 51373 Leverkusen (DE); TRAENKLE, Jens, 51373 Leverkusen (DE)
(74) Representative: Novagraaf International SA
(86) International application number: PCT/EP2022/076944
(87) International publication number: WO 2023/052401

(56) References cited:
- DE-B3- 102019 001 995
- US-A1- 2011 201 100
- US-A1- 2021 016 268
- US-B2- 6 770 441

## Description

### OBJECT OF THE INVENTION

The invention relates to a solution for monitoring or controlling bioprocesses by way of withdrawing discrete fluid samples from a reactor and performing on-line analysis of said sample using a fluidic manifold, for example, by means of a sensor based in particular on label-free biomolecular interaction analysis.

### BACKGROUND OF THE INVENTION

In-line and on-line monitoring of bioprocesses is yet limited to spectroscopic or electrochemical sensors. This is a limitation regarding the ability to quantify and monitor specific biological compounds. Current biopharmaceutical processes in development (e.g., monoclonal antibodies, recombinant proteins, vaccines, cell and gene therapies) involve a large panel of product quality attributes and process parameters that are biologically active proteins (e.g., proteins produced by the cells or present in cell culture media) which cannot be monitored on-line by existing technologies. Biosensors based on label-free protein interaction analysis or more generally bioanalytical tools may provide further valuable information for process monitoring and control.

Several off-line label-free protein biosensors have been commercialized to date based on different principles : Surface Plasmon resonance (Biacore - Cytiva), Bio-Layer Interferometry (Octet - Sartorius, Gator+ - Gator bio), Quartz Cristal Microbalance (Q-Sense - Biolin Scientific), Grating-Coupled Interferometry (Wave - Creoptix). In comparison to classical ELISA bioassays, involving a lot of manual steps and long (hours) time-to-results, those systems can perform faster protein quantitative assays and evaluate kinetic parameters of a biological interaction between an analyte of interest binding to a ligand pre-functionalized onto the biosensor. Consequently, they can be used to evaluate the biological activity of protein compounds toward a dedicated ligand.

However, those label-free biosensors are currently only used for off- or at-line analysis of bioprocesses samples (sample analysis after manual sampling, transport and preparation, potentially partly automated with liquid handlers). They are therefore not directly connected to the process ("on-line"), and thus cannot be used for automated process monitoring and control. There is a need for rapid (time-to-result <30 mins) and automated protein analysis with low sample and low reagents volume consumptions and without the help of a secondary liquid handling machine/pipetting robot.

The use of such bioanalytical tools for on-line monitoring or controlling of bioprocesses could speed up process development and increase in-process control capabilities. For example, the data obtained from the sensor (e.g. protein titer, kinetic parameters, protein activity) at defined timepoints in a process can be used as input in uni- or multivariate model(s) used to control the process.

US 20111201100 A1 discloses a disposable cell culture bioreactor manifold system for use in coupling sensors.

US 2021/016268 A1 discloses a fluid guiding module including a housing having a sensor chamber.

### SUMMARY OF THE INVENTION

The object underlying the invention is achieved by the combination of features according to the independent claims. Exemplary embodiments of the invention can be gathered from the respective dependent claims.

The invention is described in more detail without any division within the subject matter of the invention (process and system). The explanations below are intended to be applicable analogously to all of the subject matters of the invention, in either context (process or system).

The solution of the invention relies on the use of a fluidic system comprising a fluidic manifold for contacting one or more sensors or analyzers with the sample fluid derived from a process.

The process may be an industrial process and/or a bioprocess, such as a biotechnological process. The process may involve chemical or microbiological conversion of material in conjunction with the transfer of heat, mass, and energy. The process may be a batch process, e.g., a fed-batch bioprocess or perfusion process. The process may involve producing cells for use in, or to host, the product. More particularly, the cells may host the product, or the cells may be (part of) the product.

Said cell may be selected from, but is not limited to, a bacterial cell, a microbial cell, an animal cell, a mammalian cell, an insect cell, a plant cell, an algae cell, a fungus cell, and a yeast cell. Preferably, said cell is a mammalian cell, further preferably said cell is an immortalized cell line or a stem cell, more preferably said cell is a human, simian or rodent cell line, most preferably said cell line is selected from the group consisting of Chinese Hamster Ovary (CHO) cells, baby hamster kidney (BHK) cells, COS cells, mouse myeloma cells (NS0, SP2/0), human embryonic kidney (HEK) cells, human retina-derived cells (PER-C6), and human amniocyte cells (CAP cells).

Said cell further may be selected from, but is not limited to, human primary cells; embryonic, adult and induced pluripotent stem cells (iPSCs); hematopoietic stem cells (HSCs), skin stem cells (SSCs), neural stem cells (NSCs), and mesenchymal stem cells (MSCs); human immune cells, such as T cells, dendritic cells (DCs), natural killer (NK) cells, macrophages, lymphokine-activated killer (LAK) cells, cytokine-induced killer (CIK) cells, γδ T cells, and NK cells; and CAR-T cells employing synthetic antibody-based chimeric antigen receptors (CARs).

The terms "sample fluid" or "fluid sample" as used therein refer to a solution containing analytes, e. g. protein(s), to be measured by the sensor(s). Additionally, the sample fluid may include the fluid used in the process for producing the desired product. The sample fluid may include starting material for the process. More particularly, the sample fluid may include a medium and/or biological material (e.g., a cell culture medium).

The term "vessel" as used therein refers to a fermenter, a bioreactor, a sampling cup, a sampling loop or any other vessel containing the sample fluid to be analyzed.

In a first aspect, the solution of the invention is a manifold (20) adapted to provide at least one contact volume of a fluid sample for contacting with at least one sensor (5) in a system for measuring one or at least one analyte in said fluid sample, said manifold (20) comprising:
- a body (25),
- in the body (25), for each sensor (5), one fluidic cell comprising one fluid distribution channel (21) and a collecting channel (22a, 22b) in fluidic communication with the fluid distribution channel (21);
   wherein a fluid distribution tubing (30) can be fitted with or in, preferably with, the fluid distribution channel (21) so that it is emerging in the collecting channel (22a, 22b) and so that the sensor (5) can be contacted with at least a drop (10) of the fluid sample emerging at the top of the fluid distribution tubing (30) as the contact volume;
      or
   wherein the fluid distribution tubing (30) can be fitted with or in, preferably with, the fluid distribution channel (21) in fluidic connection with a measurement well (40), so the measurement well (40) can be filled with the fluid sample to create the contact volume and the sensor (5) can be dipped in the fluid sample for measurement;
- and wherein said collecting channels (22a, 22b) are plunging so the fluid sample can be collected per gravity.

In another embodiment, the solution of the present invention is a manifold (20) adapted to provide at least one fluid sample to an analyzer in a system for measuring at least one analyte in said fluid sample, said manifold (20) comprising:
- a body (25),
- in the body (25), for each fluid sample to be analyzed, one fluidic cell comprising one fluid distribution channel (21) and a collecting channel (22a, 22b) in fluidic communication with the fluid distribution channel (21);
   wherein a fluid distribution tubing (30) can be fitted with or in, preferably with, the fluid distribution channel (21), so that it is emerging in the collecting channel (22a, 22b), and wherein a sample taker can be contacted with at least a drop (10) of the fluid sample emerging at the top of the fluid distribution tubing (30) as the contact volume and can transfer an aliquot from the fluid sample to an analyzer for measurement and/or analysis:
      or
   wherein a fluid distribution tubing (30) can be fitted with or in, preferably with, the fluid distribution channel (21) in fluidic connection with a measurement well (40), so the measurement well (40) can be filled with the fluid sample, and wherein a sample taker can be immerged in the measurement well (40) so that it can take an aliquot from the fluid sample and transfer it to an analyzer for measurement and/or analysis;
- and wherein said collecting channels (22a, 22b) are plunging so the fluid sample can be collected per gravity.

In an embodiment, the manifold (20) comprises (an arrangement of) a plurality of fluidic cells, preferably in a row.

The analysis may comprise further steps requiring moving the biosensor in reaction wells using the sensor positioning system (e.g. one or more dedicated wells on a microtiter plate), said wells containing dedicated reagent(s), buffer(s) and/or regeneration solutions. In addition to addressing the fluidic cells, the sensors may spatially address dedicated well positions according to a predefined analysis protocol: for example for ligand capture or immobilization step, washing step with a buffer solution, secondary labeling step, enzymatic detection step, signal amplification step, sensor regeneration step, sensor calibration measurements or the like. In other words, the system of the invention allows signal detection with or without amplification steps.

In an embodiment, in said manifold (20) according to the present invention, the collecting channels (22a, 22b) are in fluidic connection with an analyzer, or are in fluidic connection with a storage device, e.g., a multi-well plate.

In an embodiment, the system of the invention comprises a microtiter plate comprising wells in the same arrangement as the manifold, said wells containing dedicated reagents as required by the analysis protocol. The microtiter plate may be positioned in a drawer for better handling.

In an embodiment, in said manifold (20) according to the present invention, the body (25) further comprises a main waste channel (26) or a main waste funnel in fluidic connection with the collecting channels (22a, 22b).

In an embodiment, said manifold (20) according to the present invention comprises a main distribution channel (32) in fluidic connection with the fluid distribution tubing (30) and with the one or more fluid distribution channel(s) (21).

In an embodiment of said manifold (20) according to the present invention, the analyzer may be suited to perform analysis selected from the group comprising, but not being limited to, biomolecular interaction analysis, biolayer interferometry, surface plasmon resonance (SPR) analysis, surface-enhanced Raman spectroscopy (SERS), optical spectroscopy, capillar electrophoresis, liquid chromatography (LC), high-performance liquid chromatography (HPLC), multi-angle light scattering (MALS) analysis, dynamic light scattering (DLS) analysis, mass spectrometry, cell imaging, flow cytometry and/or polymerase chain reaction (PCR).

In an embodiment, in said manifold (20) according to the present invention, said sample taker may be a needle, syringe, automate pipette or pipette robot, a tube, a flexible tube, a line and/or a hose, in fluidic connection with the analyzer.

In an embodiment, said manifold (20) according to the present invention further comprises a liquid junction, *e.g.,* a three-way valve in the fluid distribution tubing (30), connected with an alternative collecting channel, so that fluid sample can be pulled back from the contact volume and collecting channel (22a, 22b) through the fluid distribution channel (21) by hydrodynamicaly driven force, e.g., by a pump or syringe.

Said fluid sample collected by use of the liquid junction in the fluid distribution tubing (30), connected with an alternative collecting channel, may be directed to an analyzer, or to a storage device, or to a waste channel or waste bin.

The term "analyzer" as used herein refers to any device suitable for performing a measurement or analysis of any component of the fluid sample(s), in particular of any compound, product or cell produced in respective biotechnological process (for example, a recombinant protein produced by a respective host cell) which can be based on, but is not limited to, any kind of analysis such as a biomolecular interaction analysis, biolayer interferometry, surface plasmon resonance (SPR) analysis, surface-enhanced Raman spectroscopy (SERS), optical spectroscopy, capillar electrophoresis, liquid chromatography (LC), high-performance liquid chromatography (HPLC), multi-angle light scattering (MALS) analysis, mass spectrometry, cell imaging, flow cytometry and/or polymerase chain reaction (PCR).

A sensor (5), preferably an optical sensor, may be an embodiment of an analyzer, or an embodiment of a part of an analyzer.

The term "sample taker" as used herein refers to any device or means suitable for taking a fluid sample or an aliquot (a certain amount or volume) of a fluid sample and transferring it to a different component or compartment of a system, in particular to or from said manifold, said vessel, said analyzer and/or said analysis module. The sample taker may be selected from, without being limited to, a needle, syringe, automate pipette or pipette robot, tube and line.

The term "liquid junction" as used herein refers to any kind of three- or more way-valve or shutter in a flexible or inflexible tube, line or hose, preferably to a three-way valve.

In an example, the Octet RED96/Octet R is used, wherein the manifold of the present invention is positioned in alignment with the well plate (71) so that both the manifold and the well plate can be addressed by the sensor transport module.

It is obvious to the person skilled in the art that the solution of the present invention may be used for analysis of multiplexing capabilities (e.g. multiple bioreactors).

The solution of the invention is flexible and enables the evaluation of multiple analytical parameters in parallel by a single or/and multiple steps detection scheme:
- first step in-process (e.g. on-line) affinity capture of the analyte (53) by a ligand (52) pre-immobilized on the sensor (5) using the fluidic system of the invention,
- followed by signal detection with or without signal amplification by moving the sensor (5) in another position (e.g., a dedicated well on a 96 wells plate) where it is brought in contact to a solution containing dedicated reagent.

The first capture step can also be directly used for on-line protein analysis of the analyte within the fluid sample.

The biosensors may address the well plate for multiple-step analysis in dedicated buffer.

In a second aspect, the solution of the invention is a system for measuring one or more analytes (53) in discrete fluid samples from a vessel adapted to contain a fluid comprising:
- the manifold (20) as described above;
- at least one analyzer; and
- optionally an analysis module (65) comprising at least one main sampling line in fluidic connection with the vessel and one or more pumps in fluidic connection with said main sampling line(s) and adapted to selectively pump fluid in the main sampling line away from the vessel, further comprising at least one fluid distribution tubing (30) in fluidic connection with the analysis module;
and/or
comprising a means for transferring a fluid sample manually or automatically taken from the vessel into the fluidic system of the analysis module (65), wherein said means is selected from the group consisting of one or more of a syringe, a needle, a manual or automate pipette, a pipette robot, a tube, a flexible tube, a line and/or a hose.

In another embodiment, the solution of the invention is a system for measuring one or more analytes (53) in discrete fluid samples from a vessel adapted to contain a fluid comprising:
- one or more sensors (5) for the measurement of at least one analyte (53) in a contact volume of the fluid sample;
- an analysis module (65) comprising at least one main sampling line in fluidic connection with the vessel, and one or more pumps in fluidic connection with said main sampling line(s) and adapted to selectively pump fluid from the main sampling line away from the vessel;
- at least one fluid distribution tubing (30) in fluidic connection with the analysis module (65);
- a manifold (20) comprising a body (25), and in the body (25), for each sensor (5), one fluidic cell comprising one fluid distribution channel (21) and a collecting channel (22a, 22b) in fluidic communication with the fluid distribution channel (21);
- wherein the fluid distribution tubing (30) is fitted with or in, preferably with, the fluid distribution channel (21) so that it is emerging in the collecting channel (22) and so that the sensor (5) can be contacted with at least a drop (10) of the fluid sample emerging at the top of the fluid distribution tubing (30) as the contact volume;
- or
   wherein the fluid distribution tubing (30) is fitted with or in, preferably with the fluid distribution channel (21) in fluidic connection with a measurement well (40), so the measurement well (40) can be filled with the fluid sample to create the contact volume and the sensor (5) can be dipped in the contact volume for measurement;
- and wherein said collecting channels (22a, 22b) are plunging so the fluid sample can be collected per gravity.

The term "analysis module" (65) as used herein refers to a device which can be used to control and direct fluid sample flow(s) in said system for measuring analytes. Preferably, said analysis module comprises at least one main sampling line in fluidic connection with the vessel and one or more pumps in fluidic connection with said main sampling line(s) and adapted to selectively pump fluid in the main sampling line away from the vessel, and/or comprises a means for transferring a fluid sample manually or automatically taken from the vessel into its fluidic system for transfer to the analyzer, wherein said means is selected from the group consisting of one or more of a syringe, a needle, a manual or automate pipette, a pipette robot, a tube, a flexible tube, a line and/or a hose.

As used herein, the term "system" refers to a delimitable, natural or artificial structure, which consists of various components with different properties, which are viewed as a common whole due to certain ordered relationships with one another. The components of the system may be arranged in a common housing or apparatus, or the components may be arranged in different housings which are structurally and/or functionally connected to each other.

In an embodiment, the body (25) further comprises a main waste channel (26) or a main waste funnel in fluidic connection with the collecting channels (22a, 22b).

In an embodiment the sensor is an optical sensor, preferably the optical sensor is a biosensor. A "biosensor" as used herein is an analytical sensor comprising a biological reagent. Biosensors combine a detector (i.e., biorecognition elements interacting with the measured analyte) and a transducer which converts biological interactions into a measurable signal.

In a particular embodiment the sensor is an optical biosensor. The biosensor probe consisting in an optical fiber (51) based sensor as described in US 2007/0070356A1. Also, other biosensors allowing label-free bioanalyses, may be used. For example, biosensors based on surface plasmon resonance (e.g. as described in US2004/0186359), localized surface plasmon resonance (e.g. as described in WO 2017/046179Al) or focal molography (e.g. as described in EP2805149A1) could be envisioned for the application.

In an embodiment a plurality of sensors is used.

In an embodiment, an array of sensors is used. The one or more sensors are typically mounted on a support and 2 or 3 axis positioning system (also referred to as a sensor transport module) addressing the sensors to dedicated position in the manifold, sensor plate (in order to load new sensors), microtiter plate (containing reagents) or any other desired position.

For example, off-line systems for biomolecular interaction analysis using the technique of BIO-LAYER INTERFEROMETRY (BLI), wherein analyses are performed by dipping 8 aligned sensors arranged on a movable solid support and transport system in a row of 8 wells arranged in a 96-wells plate, can be used. Commercially available BLI systems include: "Octet RED96/Octet R" systems (Sartorius) and "Gator Prime/Gator plus" systems (Gator bio).

The system of the invention comprises a fluidic system dedicated for the automated delivery of the fluid sample from the process to one or more biosensors, contacting the sensors with the fluid, followed by signal detection with or without a preceding signal amplification step.

The term "fluidic system" as used therein refers to a device capable of transporting the fluid from a vessel to and from the sensor(s). The fluidic system comprises the pumps, the valves, the fluid distribution channels, the fluid distribution tubing, the optional measurement wells, the collecting channels and the waste channel or funnel.

In an embodiment, the fluid sample may be diluted with a defined volume of buffer or mixed with a reagent before filling into the manifold via the sampling line.

In an embodiment, the main sampling line and / or the fluid distribution tubing are made of PTFE, PEEK, PFA, FEP or silicon-based tubing, typically commercially available tubing with an inner diameter ranging from 0.1 to 20 mm, preferentially 0.25 to 1 mm.

The analysis module is adapted to selectively pump fluid away from the vessel into the manifold. Commercial analysis modules may be used. In an embodiment, the system may comprise one or more filters filtering the sample before it enters the manifold. Typically, such filters are placed at vent ports.

In an embodiment, high-throughput automated bioreactor and micro-bioreactor system (e.g. Sartorius AmbR 15 and AmbR 250, mp2 labs biolector XP) (50) including a liquid handling system (62) and a sample cup (64) and allowing to run multiple vessels (61) in parallel and automatically perform regular sampling and feed addition with a sampling device (63) can be used. The sampling device (63) may implement an automated pipetting system and/or carry pipet tips. Commercial analysis module (e.g. Sartorius analysis module) (65) can be used to automatically deliver the sample fluid from the sample cup (64) to the manifold (20) by fluidic connection with at least one fluid distribution tubing (30). Other bioprocess autosampling systems may be used as analysis module for automated sample collection and distribution of the sample to the manifold (20). Commercial bioprocess autosamplers comprise, but are not limited to, the following systems: Numera (Securecell), MAST (Lonza), SegFlow (Flownamics), BioPAT (Sartorius). Analysis module may require gas filters for maintenance of sterility. In an embodiment, each filter comprises a sterilizing grade gas filter with a 0.2 or 0.45 µm membrane.

One fluid distribution channel (21) in fluidic connection with its collecting channel (22) is together referred to as a "fluidic cell" of the manifold.

In an embodiment, the vessel is a fermenter, a bioreactor, a sampling cup, a sampling loop or any other vessel containing the sample fluid to be analyzed.

In an embodiment, the system comprises at least one multiparallel bioreactor system, *e.g.*, Ambr^{®} bioreactor vessels, and a biolayer interferometry sensor system, *e.g.*, Octet^{®}.

In an embodiment, a plurality of sensors is used. It is preferred that the sensors are arranged in an array, for example in a row.

In an embodiment, the manifold comprises an arrangement of a plurality of fluidic cells. For reason of compatibility with the commercial "Octet RED96/Octet R8" preferably 8 identical fluidic cells in alignment were chosen in an example.

The person skilled in the art will appreciate that the number of fluidic cells is discretionary. He will also appreciate, that the geometrical arrangement of the fluidic cells in the manifold are governed by practicability, although a manifold comprising one array of fluidic cells is preferred.

In an embodiment, the number of fluidic cells is the same as the number of sensors.

In an embodiment the geometrical arrangement of the fluidic cells is the same as the arrangement of the sensors.

In an embodiment one or more arrays of fluidic cells and / or sensors are used.

In an embodiment, the one or more fluid distribution channel (21) is in fluidic connection with a main distribution channel (32), which is in fluidic connection with the fluid distribution tubing (30).

In an embodiment, the one or more fluid distribution channel (21) is in fluidic connection with a main distribution channel (32), which is in fluidic connection with the main sampling line.

In this embodiment, it is preferred that the main distribution channel (32) is dimensioned so that the main sampling line can be fitted and maintained in the main distribution channel (32). In an example, the main distribution channel (32) is horizontal or nearly horizontal and the one or more fluid distribution channels (21) are vertical or nearly vertical. It is preferred that the fluid distribution channels (21) are identical in dimension and aligned to each other to form an array. In an embodiment the fluid distribution channels (21) are distributed along the main distribution channel (32) at regular distance. In an embodiment, the main distribution channel (32) is dimensioned so that the flow is identical or nearly identical in all the fluid distribution channels (21).

In an alternative embodiment, at least one fluid distribution tubing (30) is in fluidic connection with a analysis module (65) comprising one or more pumps and / or valves in fluidic connection with the main sampling line connected to one or more vessels. In this embodiment, it is preferred that the one or more fluid distribution channel (21) is dimensioned so that the fluid distribution tubing (30) can be fitted and maintained in the fluid distribution channel (21). In an embodiment, the one or more fluid distribution channel (21) are vertical or nearly vertical in the body.

In an embodiment, the fluid distribution tubing (30) is mounted in the fluid distribution channel (21) and maintained by a fitting (31) as a fluidic connector. Other type of fluidic connectors can be envisioned for fixing the tubing.

In an embodiment, the pumps may be peristaltic pump, diaphragm pump, piston pump, syringe pump or a combination thereof.

In an embodiment, the system comprises an automated flow control system adapted to:
(a) configure and store a sample filling configuration for the analysis module, in particular for its one or more pumps and / or valves, the sample filling configuration comprising a prescribed filling period and / or a prescribed filling volume;
(b) automatically operate said sampling system in a sampling direction away from the vessel for the filling of the sample fluid into the manifold for the prescribed using the filling configuration;
(c) configure and store a contact configuration for the analysis module, in which sample flow is stopped for a prescribed contact period, during which an analysis is automatically launched and the sensor (5) is contacted with the contact volume;
(d) automatically operate said sampling system for a prescribed contact period using the contact configuration; and / or
(e) configure and store a cleaning configuration for the system, preferred for the analysis module, the cleaning being achieved by pushing the sample fluid and / or cleaning fluid away from the sensor(s) into the collecting channels (22), said cleaning configuration comprising one or more cleaning periods and / or one or more cleaning volumes;
(f) automatically operate the system using the cleaning configuration.

In an embodiment, the system comprises an automated control system adapted to:
(a) configure said flow control system and store a sample filling configuration for the analysis module, the sample filling configuration comprising a prescribed period and / or a prescribed volume for creating the contact volume;
(b) automatically operate said sampling system in a sampling direction away from the vessel into the manifold (20) using the filling configuration;
(c) configure and store a contact configuration for the analysis module, in which sample flow is stopped for a prescribed contact period, during which an analysis is automatically launched and the sensor (5) is contacted with the contact volume, and/or the sample taker is contacted with the contact volume;
(d) automatically operate said sampling system using the contact configuration; and / or
(e) configure and store a cleaning configuration for the system, preferred for the analysis module, the cleaning being achieved by pushing the sample fluid and / or cleaning fluid away from the sensor (5) into the collecting channels (22a, 22b), said cleaning configuration comprising one or more cleaning periods and / or one or more cleaning volumes;
(f) automatically operate the system using the cleaning configuration.

The term "cleaning fluid" as used therein refers to cleaning solution and / or a buffer solution used for cleaning the fluidic system between the analysis of samples.

The term "cleaning period" refers to the time or volume needed to flush and clean the fluidic system.

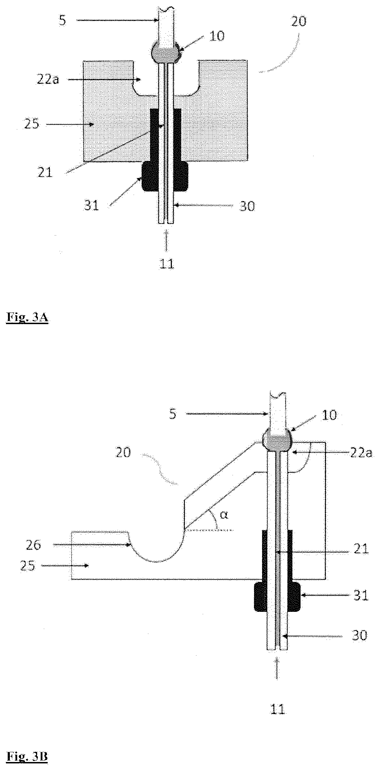

In an embodiment (Fig 3A and 3B), the fluid distribution tubing (30) is fitted in the fluid distribution channel (21) so that it is emerging in the fluid collecting well and that the sensor (5) can be contacted with at least a steady drop (10) of the fluid emerging at the top of the fluid distribution tubing (30). In this embodiment the sensor (5) is positioned so that the steady drop (10) of the fluid is emerging at the top of the fluid distribution tubing (30) and contacting with the sensor (5) is achieved during analyses.

In this embodiment, the filling period is the time required to generate the steady drop at the predefined flow rate for the sample fluid. The sample fluid is typically forwarded into the fluidic system at a flow rate ranging from 0.01 to 1 mL/min, preferred 0.1 to 0,8 mL/min, most preferred 0.5 mL/min, depending on the tubing dimensions. For the generation of the steady drop the sensors may be positioned at approximatively 2 mm of the outlet of the fluid distribution tubing. The one or more pumps of the analysis module are stopped for a prescribed contact period to prevent the fluid from flowing into the collecting channel (22) for the sensor contact period. Optimal distance, flow rate and time for the generation of drop are typically established experimentally for the specific embodiment used.

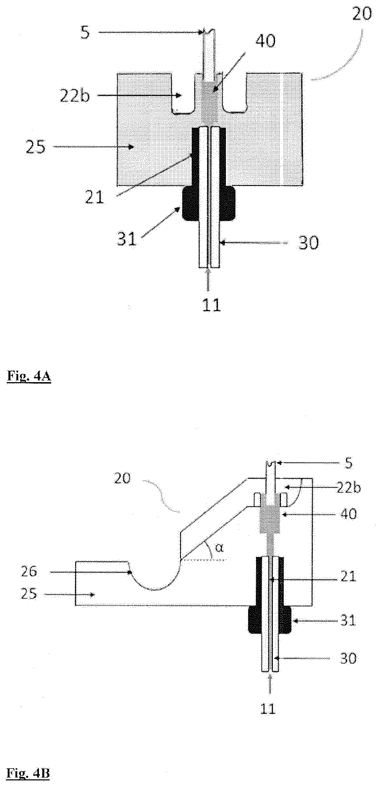

In an alternative embodiments (Fig. 4A, 4B), the fluid distribution tubing (30) is fitted in the fluid distribution channel (21) in fluidic connection with a measurement well (40), so the measuring well (40) is filled with the fluid. In this embodiment the sensor is positioned to dip into the fluid sample. In this embodiment the filling period is typically the time required to fill the measurement well (40). In this particular embodiment, the form of the measurement well (40) is preferably cylindrical with a volume from 10 to 500 µl, preferred from 100 to 400 µl, wherein the dimensions of the measurement well (40) allow the convenient positioning of the sensor or array of sensors in the fluid volume filled in the measurement well (40). Typically, the measurement well (40) shows a diameter from 1 to 5 mm, preferred 3 mm and a height from 5 to 20 mm, preferred 15 mm.

In an embodiment (Fig. 4A, 4B), the measurement well (40) is preferably overflowing into a collecting channel (22b) encompassing the measurement well (40). The collecting channel (22b) is also used for flushing with cleaning fluid.

For all embodiments described above, cleaning is conducted at the end of the contact period: the automated control system operates the one or more pumps and/ valves of the analysis module (65) to flush the fluidic system with the cleaning fluid for a predefined cleaning period, preferably in a continuous cleaning flow. The cleaning fluid flows into the collecting channel and pours out per gravity into a main waste channel or a main waste funnel in fluidic connection with the collecting channels.

In an embodiment the collecting channel (22) is plunging into the main waste channel (26) or the main waste funnel with an angle α of 5 to 80 degree, preferred 20 to 40 degree, most preferred 30 degree to the vertical (plumb line).

In an embodiment, the collecting channel (22a, 22b) may be a pipe, U-shaped or V-shaped. It is preferred U-shaped. The dimension of the collecting channel (22a, 22b) is discretionary. In a preferred embodiment the diameter of the collecting channel (22a, 22b) is from 3 to 10 mm.

It is clear to the person skilled in the art, that form and dimension of the collecting channel (22a, 22b) is discretionary as it is intended to collect the fluid drop after contact is ended and the cleaning fluid is flushed into the fluidic system.

In an embodiment, the main waste channel (26) is U-shaped or V-shaped, preferred U-shaped; the dimension of the main waste channel (26) is discretionary.

In case a main waste funnel is used, its dimensions and form are discretionary.

In an embodiment, cleaning fluid is forwarded into the fluidic system at a flow rate ranging from 1 to 10 mL/min, preferred 1 to 5 mL/min, most preferred 5 mL/min.

It is clear to the man skilled in the art, that the fluid flowing rates are typically set for controlled filling and overflowing of the measurement well (40) if used, without splashing or overflowing of the collecting channels (22a, 22b) and / or main waste channel (26) or main waste funnel.

The person skilled in the art will also appreciate, that the dimensions of a fluidic cell in the manifold as well as flow rates used for the transport of fluids in the manifold are governed by practicability and the design of the specific embodiment.

In an embodiment, the body of the manifold is made of inert plastic material. Different material can be used, encompassing but not restricted to: POM (Delrin/Acetal), Nylon, PEEK, PTFE (Teflon), Acrylonitrile butadiene styrene (ABS), Acrylic/Plexiglas (PMMA), Polycarbonate (PC), Polysulphone (PSU), Polyetherimide (PEI, Ultem), Cyclic Olefin Copolymer (COC).

In an embodiment, the body of the manifold is produced by way of CNC machining or/and molding or/and diffusion bonding. Preferably, a PEEK manifold produced by CNC machining is used.

In an embodiment, the sensors are mounted on a sensor positioning system.

In an embodiment, the system comprises a sensor positioning system, wherein an automated control system is adapted to position the sensors (5) for contacting with the fluid sample by controlling the sensor positioning system.

In an embodiment, the sensors are mounted on a 2 or 3-axis linear sensor positioning system and can be spatially addressed to dedicated positions in the fluidic cells (one sensor per fluidic cell).

In an embodiment, an automated control system is adapted to position the sensors for contacting with the fluid sample by controlling the sensor positioning system.

In a third aspect, the solution of the invention is a method for measuring analytes in discrete fluid samples from a vessel adapted to contain a fluid comprising the steps of:
a) pumping the sample fluid from the vessel into a manifold (20) as described above to create a contact volume of fluid sample in the fluidic cells of the manifold (20);
   or
   transferring the sample fluid taken from the vessel into a manifold (20) as described above by transferring the fluid sample manually or automatically taken from the vessel into the fluidic system of the manifold (20), wherein said transfer may be employed by using one or more of a syringe, a needle, a manual or automate pipette, a pipette to create a contact volume of fluid sample in the fluidic cells of the manifold (20);
b) contacting the sensor (5) with the contact volume and conducting measurement for a contact period;
   or
   contacting a sample taker with at least a drop (10) of the fluid sample emerging at the top of the fluid distribution tubing (30) as the contact volume and transferring an aliquot from the fluid sample to an analyzer for conducting measurement and/or analysis;
      or
   immerging a sample taker into the measurement well (40), taking an aliquot from the fluid sample with the sample taker, and transferring an aliquot from the fluid sample to an analyzer for conducting measurement and/or analysis;
c) transferring or pushing the fluid sample into the collecting channels (22a, 22b) and eliminating the fluid sample per gravity;
   or
   transferring or pushing the fluid sample into the collecting channels (22a, 22b) which are in fluidic connection with an analyzer for further measurement, or collecting the fluid sample per gravity into storage device, *e.g.,* a multi-well plate;
d) outputting and / or forwarding measurement on a user interface or to an on-line process monitoring device or a process control system.

A further object of the invention is a method for measuring analytes in discrete fluid samples from a vessel adapted to contain a fluid comprising the steps of:
a) pumping the sample fluid from the vessel into a manifold (20) as described above to create a contact volume of fluid sample in the fluidic cells of the manifold (20);
b) contacting the sensor (5) with the contact volume and conducting measurement for a contact period;
c) pushing the fluid sample into the collecting channels (22a, 22b) and eliminating the fluid sample per gravity;
d) outputting and / or forwarding measurement on a user interface or to an on-line process monitoring device.

The method may further comprise cleaning the system by way of pumping sample fluid and / or cleaning fluid through the fluidic cell into the collecting channels (22a, 22b).

In an embodiment, the method steps are reiterated in a timely defined manner so that in-process samples can be analyzed automatically.

The method of the invention is particularly useful for measuring analytes in a biological process, in particular for on-line process monitoring.

The output of the method of the invention may be used for process control. In particular, the output of the sample analysis can be used as an input variable in a process control model.

The solution of the invention solves several issues:
a) low sample volume consumption;
b) low reagent volume consumption (the reagents are kept in the well plate and re-used for multiple analysis);
c) Automated sample processing (transport, dilution, reagent mixing) and analysis with a biosensor, without the need of external microtiter plate handling robot.

This configuration offers a very low (≤50 uL) sample volume consumption per analysis and is therefore advantageous for monitoring small-scale bioprocesses.

Additionally, the system may comprise different biosensors for parallel evaluation of different attributes related to one or several analytes. This configuration allows minimum reagent and calibrator consumption as the reagents placed in the well plate can be used for multiple analyses.

The underlying sensing principle is based on Bio-layer interferometry (BLI), allowing for label-free quantification of analytes with time of analysis ranging from seconds to minutes, potentially allowing in-process control based on analysis.

Analytical measurements from the biosensor performed at given timepoint of the process may also be used as an input in a process control model used to determine process outputs in a biopharmaceutical and/or biological process. A process output may be a key performance indicator (e.g., product titer), a product specific (critical) quality attribute, CQA, (e.g., glycosylation profile), or a critical process parameter, CPP (e.g. concentration and/or biological activity of a given protein added into the culture medium). The method may further comprise initiating a control action via a process control device in order to optimize the process output value. The control action may include increasing or decreasing supply of a nutrient. For example, WO2020173844A1 (Multivariate process chart to control a process to produce a chemical, pharmaceutical, biopharmaceutical and/or biological product) and WO2017199006A1 (automated bioprocess development) describe the use of such process control models in bioprocesses.

The use of terms "a" and "an" and "the" and similar referents in the context of describing the invention (especially in the context of the following claims) are to be construed to cover both the singular and the plural, unless otherwise indicated herein or clearly contradicted by context. The terms "comprising", "including", "having", and "containing" are to be construed as open-ended terms (i.e. meaning "including but not limited to") unless otherwise noted.

The use of any and all examples, or exemplary language (e.g., "such as") provided herein, is intended merely to better illuminate the invention and does not pose a limitation on the scope of the invention unless otherwise claimed. No language in the specifications should be constructed as indicating any non-claimed element as essential to the practice of the invention.

Preferred embodiments of this invention are described, including the best mode known to the inventors for carrying out the invention. Variations of those preferred embodiments can become apparent to those of ordinary skilled artisans to employ such variations as appropriate, and the inventors intend for the inventions to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### Examples

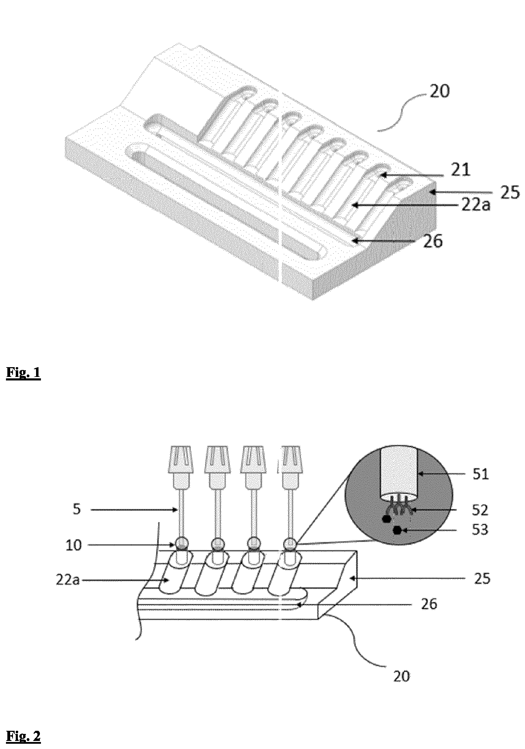

Fig. 1 shows a 3-dimensional side view from above of a manifold with an array of fluidic cells according to the embodiment of Fig. 4.
Fig. 2 shows a 3-dimensional side view of the system of the invention wherein the sensors (5) are contacting with a drop (10) generated in the fluidic cells. The enlargement shows the principle of ligand (52) capture of the analyte (53) at the tip of the sensors (5).
Fig. 3A and 3B shows section diagrams of a fluidic cell, wherein a stable drop (10) is generated for sample measurement.
Fig. 4A and 4B shows section diagrams of a fluidic cell, wherein a measurement well (40) is used for sample measurement.

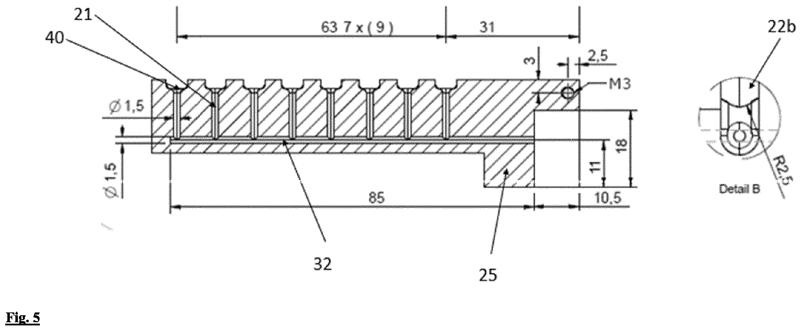

Fig. 5 shows construction diagrams of a manifold (20) according to of a particular embodiment of the system of the invention wherein a main distribution channel (32) in fluid connection with fluid distribution channels (21) is used.
Fig. 6 shows a side view of a system of the invention wherein manifold (20) is implemented within an Octet RED96/Octet R system connected to an AmbR high-throughput automated bioreactor. The analysis module (65) is used to address fluid samples from a sample cup (64) to the manifold (20).
Fig. 7 shows the application of the invention to perform on-line monitoring of antibody titers in a vessel by bio-layer interferometry with the Octet RED96 system (70).
Fig. 8A shows the raw data obtained by the Octet RED96 system for all conducted analysis according to example 2.
Fig 8B shows the resulting outputs (antibody titers) evaluated over the 14h period by the Octet system and the theoretical antibody concentration in the vessel according to the concentration of the stock solution and the pump flow rate according to example 2.

### References:

5 sensor
51 optical fiber
52 ligand
53 analyte
10 drop
11 Sample
20 manifold
21 fluid distribution channel
22a, 22b collecting channel
25 body
26 main waste channel
30 fluid distribution tubing
31 fitting
32 main distribution channel
40 measurement well
50 automated bioreactor system
61 vessel
62 liquid handling system
63 sampling device
64 sample cup
65 analysis module
66 syringe pump
67 stock vessel
68 pumps
69 medium
70 bio-layer interferometry analyzer - Octet R
71 well plate
72 medium vessel

### Example 1 - Manifold with stable sample drop

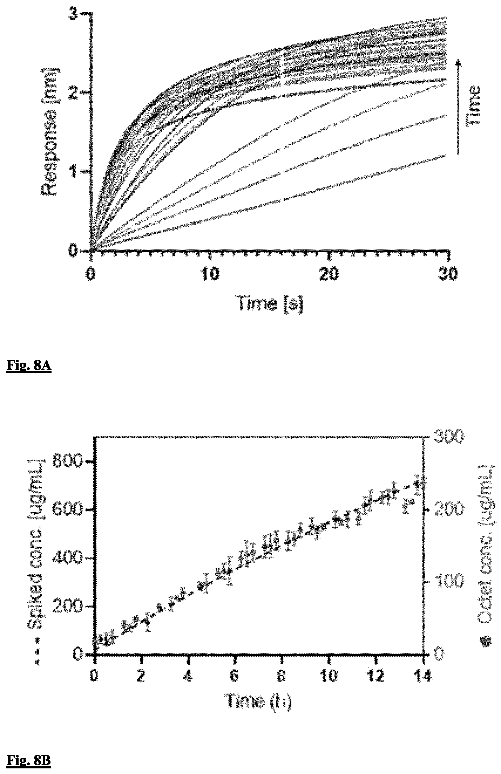

Fig. 8 shows an example of a production diagram for the body of a manifold with fluidic cells according to the embodiment of Fig. 3 with exemplary dimensions. In an example a manifold (20) comprising 8 fluidic cells was produced by machining PEEK. The manifold, the fluid distribution tubing (30) (PTFE Teflon) is fitted in each fluid distribution channel (21) so that it is emerging in the fluid collecting well (22).

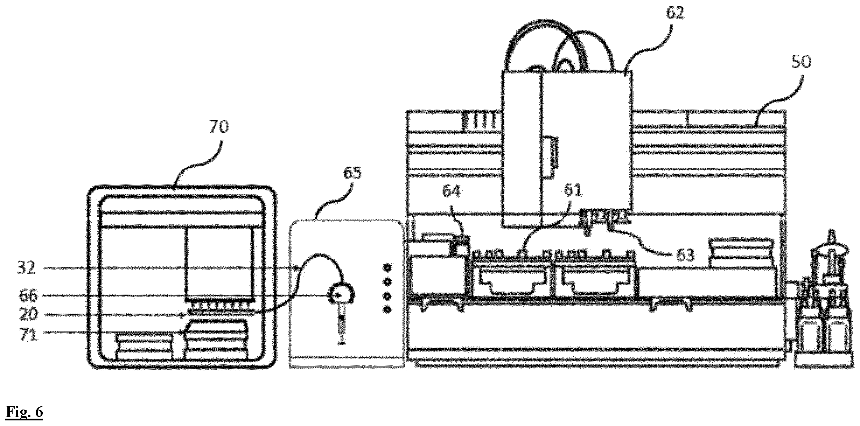

Fig. 6 shows the implementation of the manifold within an Octet RED96/Octet R system (70) connected to an AmbR 15 high-throughput automated bioreactor system (50), equipped with a liquid handling system (62) that can automatically dispense and extracts sample fluid from the vessels (61), and can address them to the sample cup (64) of the analysis module (65). The analysis module (65) is used to automatically address fluid samples from a sample cup (64) to the manifold (20), using one syringe pump (66) for the provision of the sample. The manifold (20) was arranged in an Octet RED96/Octet R (70), comprising the sensors (5) and a well plate in a 96-wells format (71).

For the generation of the stable drop (10) the Octet RED96/Octet R was configured to position the sensors at a distance between the sensor and the outlet of the fluid distribution tubing (30) of approximatively 2 mms and the analysis module (65) was configured to push the adequate volume of fluidic sample into the fluid distribution tubing (30) over a predetermined distance.

### Example 2 - On-line monitoring of antibody titers

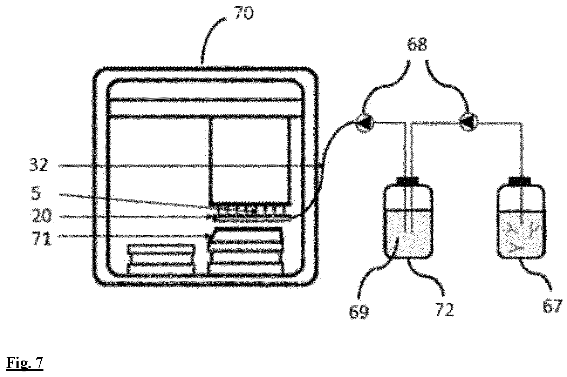

Fig. 7 shows the application of the invention to perform on-line monitoring of antibody titers in a vessel by bio-layer interferometry with the Octet RED96 system (70). Biosensors immobilized with protein A were used. Protein A can bind with strong affinity to the Fc portion of antibodies and is thus classically used as a ligand (52) for antibody quantitation. Regular increase of antibody titer in the vessel (61) was obtained by injecting antibody solution from a second stock vessel (67) of known concentration with the help of a pump (68) at a constant flow rate. The vessel initially contained only a defined volume of medium without antibodies. Additionally, the fluidic system comprised a second pump (68) in order to automatically and timely (every 15 mins) address fluid samples from the vessel to the manifold (20) placed in the Octet RED96 system, where the sensors (5) were automatically addressed for the given contact period in order to perform analysis.

Antibody quantitative assays were automatically performed in duplicate (2 sensors) every 15 mins, right after addressing fluid samples from the vessel (61), during a 14h time period. The contact period between protein A biosensors and the fluid samples was fixed to 30s. Calibration were performed in the well plate (71) every 1 h. Fig. 8A shows the raw data obtained by the Octet RED96 system for all conducted analysis. Fig 8B shows the resulting outputs (antibody titers) evaluated over the 14h period by the Octet system and the theoretical antibody concentration (dotted line) in the vessel according to the concentration of the stock solution and the pump flow rate.

## Claims

1. A manifold (20) adapted to provide at least one contact volume of a fluid sample for contacting with at least one sensor (5) in a system for measuring at least one analyte in said fluid sample, said manifold (20) comprising:
- a body (25),
- in the body (25), for each sensor (5), one fluidic cell comprising one fluid distribution channel (21) and a collecting channel (22a, 22b) in fluidic communication with the fluid distribution channel (21);
wherein a fluid distribution tubing (30) can be fitted with the fluid distribution channel (21) so that it is emerging in the collecting channel (22a, 22b) and so that the sensor (5) can be contacted with at least a drop (10) of the fluid sample emerging at the top of the fluid distribution tubing (30) as the contact volume;
or
wherein the fluid distribution tubing (30) can be fitted with the fluid distribution channel (21) in fluidic connection with a measurement well (40), so the measurement well (40) can be filled with the fluid sample to create the contact volume and the sensor (5) can be dipped in the fluid sample for measurement;
- and wherein said collecting channels (22a, 22b) are plunging so the fluid sample can be collected per gravity.

2. A manifold (20) adapted to provide at least one fluid sample to an analyzer in a system for measuring at least one analyte in said fluid sample, said manifold (20) comprising:
- a body (25),
- in the body (25), for each fluid sample to be analyzed, one fluidic cell comprising one fluid distribution channel (21) and a collecting channel (22a, 22b) in fluidic communication with the fluid distribution channel (21);
wherein a fluid distribution tubing (30) can be fitted with the fluid distribution channel (21), so that it is emerging in the collecting channel (22a, 22b), and wherein a sample taker can be contacted with at least a drop (10) of the fluid sample emerging at the top of the fluid distribution tubing (30) as the contact volume and can transfer an aliquot from the fluid sample to an analyzer for measurement and/or analysis;
or
wherein a fluid distribution tubing (30) can be fitted with the fluid distribution channel (21) in fluidic connection with a measurement well (40), so the measurement well (40) can be filled with the fluid sample, and wherein a sample taker can be immerged in the measurement well (40) so that it can take an aliquot from the fluid sample and transfer it to an analyzer for measurement and/or analysis;
- and wherein said collecting channels (22a, 22b) are plunging so the fluid sample can be collected per gravity.

3. The manifold (20) according to claim 1 or 2, comprising a plurality of fluidic cells.

4. The manifold (20) according to any one of claims 1-3, wherein the collecting channels (22a, 22b) are in fluidic connection with an analyzer, or are in fluidic connection with a storage device, *e.g.*, a multi-well plate.

5. The manifold (20) according to any one of claims 1-3, wherein the body (25) further comprises a main waste channel (26) or a main waste funnel in fluidic connection with the collecting channels (22a, 22b).

6. The manifold (20) according to any one of the preceding claims, comprising a main distribution channel (32) in fluidic connection with the fluid distribution tubing (30) and with the one or more fluid distribution channel (21).

7. The manifold (20) according to any of claims 2-5, wherein the analyzer may be suited to perform analysis selected from the group comprising biomolecular interaction analysis, biolayer interferometry, surface plasmon resonance (SPR) analysis, surface-enhanced Raman spectroscopy (SERS), optical spectroscopy, capillar electrophoresis, liquid chromatography (LC), high-performance liquid chromatography (HPLC), multi-angle light scattering (MALS) analysis, dynamic light scattering (DLS) analysis, mass spectrometry, cell imaging, flow cytometry, and/or polymerase chain reaction (PCR).

8. The manifold (20) according to any of claims 2-6, wherein the sample taker may be a needle, syringe, automate pipette or pipette robot, a tube, a flexible tube, a line and/or a hose in fluidic connection with the analyzer.

9. The manifold (20) according to any one of the preceding claims, further comprising a liquid junction, *e.g*., a three-way valve in the fluid distribution tubing (30), connected with an alternative collecting channel, so that fluid sample can be pulled back from the contact volume, drop (10) or measurement well (40) through the fluid distribution channel (21) by hydrodynamicaly driven force, *e.g.,* by a pump or syringe, preferably
wherein the fluid sample collected by use of the liquid junction in the fluid distribution tubing (30), connected with an alternative collecting channel, may be directed to an analyzer, or to a storage device, or to a waste channel or waste bin.

10. A system for measuring one or more analytes (53) in discrete fluid samples from a vessel adapted to contain a fluid comprising:
- the manifold (20) according to any one of claims 1-9;
- at least one analyzer; and
- optionally an analysis module (65) comprising at least one main sampling line in fluidic connection with the vessel and one or more pumps in fluidic connection with said main sampling line(s) and adapted to selectively pump fluid in the main sampling line away from the vessel, further comprising at least one fluid distribution tubing (30) in fluidic connection with the analysis module;
and/or
comprising a means for transfering a fluid sample manually or automaticaly taken from the vessel into the fluidic system of the analysis module (65), wherein said means is selected from the group consisting of one or more of a syringe, a needle, a manual or automate pipette, a pipette robot, a tube, a flexible tube, a line and/or a hose, preferably
wherein said analyzer is any device suitable for performing a measurement or analysis, preferably wherein said analysis is a biomolecular interaction analysis, biolayer interferometry, surface plasmon resonance (SPR) analysis, surface-enhanced Raman spectroscopy (SERS), optical spectroscopy, capillar electrophoresis, liquid chromatography (LC), high-performance liquid chromatography (HPLC), multi-angle light scattering (MALS) analysis, mass spectrometry, cell imaging, flow cytometry and/or polymerase chain reaction (PCR).

11. A system for measuring one or more analytes (53) in discrete fluid samples from a vessel adapted to contain a fluid comprising:
- one or more sensors (5) for the measurement of at least one analyte (53) in a contact volume of the fluid sample;
- an analysis module (65) comprising at least one main sampling line in fluidic connection with the vessel and one or more pumps in fluidic connection with said main sampling line(s) and adapted to selectively pump fluid from in the main sampling line away from the vessel;
- at least one fluid distribution tubing (30) in fluidic connection with the analysis module;
- a manifold (20) according to any one of claims 1 and 3-9 when dependent on claim 1.

12. The system of claim 11, wherein the sensor is an optical sensor or a biosensor.

13. The system according to any one of claims 11-12, wherein a plurality of sensors is used.

14. The system according to any one of claims 10-13, wherein the system comprises at least one multiparallel bioreactor system, e.g., Ambr^{®} bioreactor vessels, and an biolayer interferometry sensor system, e.g., Octet^{®}.

15. The system according to any one of claims 10-14, further comprising an automated flow control system adapted to:
(a) configure said flow control system and store a sample filling configuration for the analysis module, the sample filling configuration comprising a prescribed period and / or a prescribed volume for creating the contact volume;
(b) automatically operate said sampling system in a sampling direction away from the vessel into the manifold (20) using the filling configuration;
(c) configure and store a contact configuration for the analysis module, in which sample flow is stopped for a prescribed contact period, during which an analysis is automatically launched and the sensor (5) is contacted with the contact volume, and/or the sample taker is contacted with the contact volume;
(d) automatically operate said sampling system using the contact configuration; and / or
(e) configure and store a cleaning configuration for the system, preferred for the analysis module, the cleaning being achieved by pushing the sample fluid and / or cleaning fluid away from the sensor (5) into the collecting channels (22a, 22b), said cleaning configuration comprising one or more cleaning periods and / or one or more cleaning volumes;
(f) automatically operate the system using the cleaning configuration.

16. A method for measuring analytes in discrete fluid samples from a vessel adapted to contain a fluid comprising the steps of:
a) pumping the sample fluid from the vessel into a manifold (20) according to any one of the claims 1 and 3 to 9 to create a contact volume of fluid sample in the fluidic cells of the manifold (20);
or
transferring the sample fluid taken from the vessel into a manifold (20) according to any one of claims 2 to 9 by transferring the fluid sample manually or automatically taken from the vessel into the fluidic system of the manifold (20), wherein said transfer may be employed by using one or more of a syringe, a needle, a manual or automate pipette, a pipette to create a contact volume of fluid sample in the fluidic cells of the manifold (20);
b) contacting the sensor (5) with the contact volume and conducting measurement for a contact period;
or
contacting a sample taker with at least a drop (10) of the fluid sample emerging at the top of the fluid distribution tubing (30) as the contact volume and transferring an aliquot from the fluid sample to an analyzer for conducting measurement and/or analysis;
or
immerging a sample taker into the measurement well (40), taking an aliquot from the fluid sample with the sample taker, and transferring an aliquot from the fluid sample to an analyzer for conducting measurement and/or analysis;
c) transferring the fluid sample into the collecting channels (22a, 22b) and eliminating the fluid sample per gravity;
or
transferring the fluid sample into the collecting channels (22a, 22b) which are in fluidic connection with an analyzer for further measurement, or collecting the fluid sample per gravity into storage device, *e.g*., a multi-well plate;
d) outputting and / or forwarding measurement on a user interface or to an on-line process monitoring device or a process control system.

## Patentansprüche

1. Ein Verteiler (20), der dazu geeignet ist, mindestens ein Kontaktvolumen einer Flüssigkeitsprobe für den Kontakt mit mindestens einem Sensor (5) in einem System zur Messung mindestens eines Analyten in der Flüssigkeitsprobe bereitzustellen, wobei der Verteiler (20) umfasst:
- einen Körper (25),
- im Körper (25), für jeden Sensor (5), eine Fluidikzelle, die einen Fluidverteilungskanal (21) und einen Sammelkanal (22a, 22b) in fluidischer Verbindung mit dem Fluidverteilungskanal (21) umfasst;
wobei ein Fluidverteilungsschlauch (30) mit dem Fluidverteilungskanal (21) so eingebaut werden kann, dass er im Sammelkanal (22a, 22b) mündet und der Sensor (5) mit mindestens einem Tropfen (10) der Flüssigkeitsprobe, der an der Spitze des Fluidverteilungsschlauchs (30) erscheint, als Kontaktvolumen in Kontakt gebracht werden kann;
oder
wobei der Fluidverteilungsschlauch (30) mit dem Fluidverteilungskanal (21) in fluidischer Verbindung mit einem Messbrunnen (40) so eingebaut werden kann, dass der Messbrunnen (40) mit der Flüssigkeitsprobe gefüllt werden kann, um das Kontaktvolumen zu erzeugen, und der Sensor (5) zur Messung in die Flüssigkeitsprobe getaucht werden kann;
- und wobei die Sammelkanäle (22a, 22b) abfallend ausgeführt sind, sodass die Flüssigkeitsprobe durch Schwerkraft gesammelt werden kann.

2. Ein Verteiler (20), der dazu geeignet ist, mindestens eine Flüssigkeitsprobe in einem System zur Messung mindestens eines Analyten in der Flüssigkeitsprobe einem Analysator bereitzustellen, wobei der Verteiler (20) umfasst:
- einen Körper (25),
- im Körper (25), für jede zu analysierende Flüssigkeitsprobe, eine Fluidikzelle, die einen Fluidverteilungskanal (21) und einen Sammelkanal (22a, 22b) in fluidischer Verbindung mit dem Fluidverteilungskanal (21) umfasst;
wobei ein Fluidverteilungsschlauch (30) mit dem Fluidverteilungskanal (21) so eingebaut werden kann, dass er im Sammelkanal (22a, 22b) mündet, und wobei ein Probenehmer mit mindestens einem Tropfen (10) der Flüssigkeitsprobe, der an der Spitze des Fluidverteilungsschlauchs (30) erscheint, als Kontaktvolumen in Kontakt gebracht werden kann und ein Aliquot der Flüssigkeitsprobe zur Messung und/oder Analyse an einen Analysator übertragen kann;
oder
wobei der Fluidverteilungsschlauch (30) mit dem Fluidverteilungskanal (21) in fluidischer Verbindung mit einem Messbrunnen (40) so eingebaut werden kann, dass der Messbrunnen (40) mit der Flüssigkeitsprobe gefüllt werden kann, und wobei ein Probenehmer in den Messbrunnen (40) eingetaucht werden kann, sodass er ein Aliquot aus der Flüssigkeitsprobe entnehmen und zur Messung und/oder Analyse an einen Analysator übertragen kann;
- und wobei die Sammelkanäle (22a, 22b) abfallend ausgeführt sind, sodass die Flüssigkeitsprobe durch Schwerkraft gesammelt werden kann.

3. Der Verteiler (20) nach Anspruch 1 oder 2, der eine Vielzahl von Fluidikzellen umfasst.

4. Der Verteiler (20) nach einem der Ansprüche 1 bis 3, wobei die Sammelkanäle (22a, 22b) in fluidischer Verbindung mit einem Analysator oder in fluidischer Verbindung mit einer Speichervorrichtung, z. B. einer Mehrwellplatte, stehen.

5. Der Verteiler (20) nach einem der Ansprüche 1 bis 3, wobei der Körper (25) ferner einen Hauptabfallkanal (26) oder einen Hauptabfalltrichter in fluidischer Verbindung mit den Sammelkanälen (22a, 22b) umfasst.

6. Der Verteiler (20) nach einem der vorangehenden Ansprüche, der einen Hauptverteilungskanal (32) in fluidischer Verbindung mit dem Fluidverteilungsschlauch (30) und mit dem einen oder den mehreren Fluidverteilungskanälen (21) umfasst.

7. Der Verteiler (20) nach einem der Ansprüche 2 bis 5, wobei der Analysator zur Durchführung einer Analyse geeignet sein kann, die aus der Gruppe ausgewählt wird, die umfasst: biomolekulare Interaktionsanalyse, Bioschicht-Interferometrie, Oberflächenplasmonenresonanz-(SPR-) Analyse, oberflächenverstärkte Raman-Spektroskopie (SERS), optische Spektroskopie, Kapillarelektrophorese, Flüssigchromatographie (LC), Hochleistungsflüssigchromatographie (HPLC), Mehrwinkel-Lichtstreuungs-(MALS-)Analyse, dynamische Lichtstreuungs-(DLS-)Analyse, Massenspektrometrie, Zellbildgebung, Durchflusszytometrie und/oder Polymerasekettenreaktion (PCR).

8. Der Verteiler (20) nach einem der Ansprüche 2 bis 6, wobei der Probenehmer eine Nadel, eine Spritze, eine automatische Pipette oder ein Pipettierroboter, ein Schlauch, ein flexibler Schlauch, eine Leitung und/oder ein Schlauch in fluidischer Verbindung mit dem Analysator sein kann.

9. Der Verteiler (20) nach einem der vorangehenden Ansprüche, der ferner eine Flüssigkeitsweiche umfasst, z. B. ein Dreiwegeventil im Fluidverteilungsschlauch (30), das mit einem alternativen Sammelkanal verbunden ist, sodass die Flüssigkeitsprobe durch eine hydrodynamisch angetriebene Kraft, z. B. durch eine Pumpe oder eine Spritze, vorzugsweise
wobei die unter Verwendung der Flüssigkeitsweiche im Fluidverteilungsschlauch (30) gesammelte Flüssigkeitsprobe, die mit einem alternativen Sammelkanal verbunden ist, zu einem Analysator, zu einer Speichervorrichtung oder zu einem Abfallkanal oder Abfallbehälter geleitet werden kann, aus dem Kontaktvolumen, dem Tropfen (10) oder dem Messbrunnen (40) durch den Fluidverteilungskanal (21) zurückgezogen werden kann.

10. Ein System zur Messung eines oder mehrerer Analyten (53) in diskreten Flüssigkeitsproben aus einem Behälter, der zur Aufnahme einer Flüssigkeit geeignet ist, umfassend:
- den Verteiler (20) nach einem der Ansprüche 1 bis 9;
- mindestens einen Analysator; und
- wahlweise ein Analysemodul (65), das mindestens eine Hauptprobenahmeleitung in fluidischer Verbindung mit dem Behälter und eine oder mehrere Pumpen in fluidischer Verbindung mit der/den Hauptprobenahmeleitung(en) umfasst und dazu geeignet ist, Fluid in der Hauptprobenahmeleitung vom Behälter weg selektiv zu pumpen, das ferner mindestens einen Fluidverteilungsschlauch (30) in fluidischer Verbindung mit dem Analysemodul umfasst;
und/oder
ein Mittel zum manuellen oder automatischen Übertragen einer aus dem Behälter entnommenen Flüssigkeitsprobe in das Fluidik-System des Analysemoduls (65) umfassend, wobei das Mittel aus der Gruppe ausgewählt wird, die aus einer oder mehreren Spritzen, einer Nadel, einer manuellen oder automatischen Pipette, einem Pipettierroboter, einem Schlauch, einem flexiblen Schlauch, einer Leitung und/oder einem Schlauch besteht, vorzugsweise
wobei der Analysator ein beliebiges Gerät ist, das zur Durchführung einer Messung oder Analyse geeignet ist, vorzugsweise wobei die Analyse eine biomolekulare Interaktionsanalyse, eine Bioschicht-Interferometrie, eine Oberflächenplasmonenresonanz-(SPR-)Analyse, eine oberflächenverstärkte Raman-Spektroskopie (SERS), eine optische Spektroskopie, eine Kapillarelektrophorese, eine Flüssigchromatographie (LC), eine Hochleistungsflüssigchromatographie (HPLC), eine Mehrwinkel-Lichtstreuungs-(MALS-)Analyse, eine Massenspektrometrie, eine Zellbildgebung, eine Durchflusszytometrie und/oder eine Polymerasekettenreaktion (PCR) ist.

11. Ein System zur Messung eines oder mehrerer Analyten (53) in diskreten Flüssigkeitsproben aus einem Behälter, der zur Aufnahme einer Flüssigkeit geeignet ist, umfassend:
- einen oder mehrere Sensoren (5) zur Messung mindestens eines Analyten (53) in einem Kontaktvolumen der Flüssigkeitsprobe;
- ein Analysemodul (65), das mindestens eine Hauptprobenahmeleitung in fluidischer Verbindung mit dem Behälter und eine oder mehrere Pumpen in fluidischer Verbindung mit der/den Hauptprobenahmeleitung(en) umfasst und dazu geeignet ist, Fluid in der Hauptprobenahmeleitung vom Behälter weg selektiv zu pumpen;
- mindestens einen Fluidverteilungsschlauch (30) in fluidischer Verbindung mit dem Analysemodul;
- einen Verteiler (20) nach einem der Ansprüche 1 und 3 bis 9, wenn abhängig von Anspruch 1.

12. Das System nach Anspruch 11, wobei der Sensor ein optischer Sensor oder ein Biosensor ist.

13. Das System nach einem der Ansprüche 11 bis 12, wobei eine Vielzahl von Sensoren verwendet wird.

14. Das System nach einem der Ansprüche 10 bis 13, wobei das System mindestens ein multiparalleles Bioreaktorsystem, z. B. Ambr^{®}-Bioreaktor-Behälter, und ein Bioschicht-Interferometrie-Sensorsystem, z. B. Octet^{®}, umfasst.

15. Das System nach einem der Ansprüche 10 bis 14, das ferner ein automatisches Durchflussregelungssystem umfasst, das dazu geeignet ist:
(a) das Durchflussregelungssystem zu konfigurieren und eine Probenbefüllungskonfiguration für das Analysemodul zu speichern, wobei die Probenbefüllungskonfiguration einen vorgeschriebenen Zeitraum und/oder ein vorgeschriebenes Volumen zur Erzeugung des Kontaktvolumens umfasst;
(b) das Probenahme-System automatisch in einer Probenahmerichtung vom Behälter in den Verteiler (20) unter Verwendung der Befüllungskonfiguration zu betreiben;
(c) eine Kontaktkonfiguration für das Analysemodul zu konfigurieren und zu speichern, bei der der Probenfluss für einen vorgeschriebenen Kontaktzeitraum gestoppt wird, während dessen eine Analyse automatisch gestartet und der Sensor (5) mit dem Kontaktvolumen in Kontakt gebracht wird und/oder der Probenehmer mit dem Kontaktvolumen in Kontakt gebracht wird;
(d) das Probenahme-System unter Verwendung der Kontaktkonfiguration automatisch zu betreiben; und/oder
(e) eine Reinigungskonfiguration für das System, vorzugsweise für das Analysemodul, zu konfigurieren und zu speichern, wobei die Reinigung durch Drücken der Flüssigkeitsprobe und/oder der Reinigungsflüssigkeit vom Sensor (5) in die Sammelkanäle (22a, 22b) erreicht wird, wobei die Reinigungskonfiguration einen oder mehrere Reinigungszeiträume und/oder ein oder mehrere Reinigungsvolumina umfasst;
(f) das System unter Verwendung der Reinigungskonfiguration automatisch zu betreiben.

16. Ein Verfahren zur Messung von Analyten in diskreten Flüssigkeitsproben aus einem Behälter, der zur Aufnahme einer Flüssigkeit geeignet ist, umfassend die Schritte:
a) Pumpen der Flüssigkeitsprobe aus dem Behälter in einen Verteiler (20) nach einem der Ansprüche 1 und 3 bis 9, um ein Kontaktvolumen der Flüssigkeitsprobe in den Fluidikzellen des Verteilers (20) zu erzeugen;
oder
Übertragen der aus dem Behälter entnommenen Flüssigkeitsprobe in einen Verteiler (20) nach einem der Ansprüche 2 bis 9 durch manuelles oder automatisches Übertragen der Flüssigkeitsprobe aus dem Behälter in das Fluidik-System des Verteilers (20), wobei das Übertragen durch Verwendung einer oder mehrerer Spritzen, einer Nadel, einer manuellen oder automatischen Pipette, einer Pipette zur Erzeugung eines Kontaktvolumens der Flüssigkeitsprobe in den Fluidikzellen des Verteilers (20) erfolgen kann;
b) Inkontaktbringen des Sensors (5) mit dem Kontaktvolumen und Durchführen einer Messung für einen Kontaktzeitraum;
oder
Inkontaktbringen eines Probennehmers mit mindestens einem Tropfen (10) der Flüssigkeitsprobe, der an der Spitze des Fluidverteilungsschlauchs (30) als Kontaktvolumen erscheint, und Übertragen eines Aliquots der Flüssigkeitsprobe an einen Analysator zur Durchführung der Messung und/oder Analyse;
oder
Eintauchen eines Probennehmers in den Messbrunnen (40), Entnehmen eines Aliquots aus der Flüssigkeitsprobe mit dem Probenehmer und Übertragen eines Aliquots der Flüssigkeitsprobe an einen Analysator zur Durchführung der Messung und/oder Analyse;
c) Überführen der Flüssigkeitsprobe in die Sammelkanäle (22a, 22b) und Beseitigen der Flüssigkeitsprobe durch Schwerkraft;
oder
Überführen der Flüssigkeitsprobe in die Sammelkanäle (22a, 22b), die in fluidischer Verbindung mit einem Analysator für weitere Messungen stehen, oder Sammeln der Flüssigkeitsprobe durch Schwerkraft in einer Speichervorrichtung, z. B. einer Mehrwellplatte;
d) Ausgeben und/oder Weiterleiten der Messung auf eine Benutzeroberfläche oder an ein Online-Prozessüberwachungsgerät oder ein Prozessleitsystem.

## Revendications

1. Un collecteur (20) adapté pour fournir au moins un volume de contact d'un échantillon de fluide pour la mise en contact avec au moins un capteur (5) dans un système pour mesurer au moins un analyte dans ledit échantillon de fluide, ledit collecteur (20) comprenant :
- un corps (25),
- dans le corps (25), pour chaque capteur (5), une cellule fluidique comprenant un canal de distribution de fluide (21) et un canal de collecte (22a, 22b) en communication fluidique avec le canal de distribution de fluide (21) ;
dans lequel un tube de distribution de fluide (30) peut être ajusté avec le canal de distribution de fluide (21) de sorte qu'il émerge dans le canal de collecte (22a, 22b) et de sorte que le capteur (5) peut être mis en contact avec au moins une goutte (10) de l'échantillon de fluide émergeant au sommet du tube de distribution de fluide (30) en tant que volume de contact ;
ou
dans lequel le tube de distribution de fluide (30) peut être ajusté avec le canal de distribution de fluide (21) en connexion fluidique avec un puits de mesure (40), de sorte que le puits de mesure (40) peut être rempli avec l'échantillon de fluide pour créer le volume de contact et le capteur (5) peut être plongé dans l'échantillon de fluide pour la mesure ;
- et dans lequel lesdits canaux de collecte (22a, 22b) sont plongeants de sorte que l'échantillon de fluide peut être collecté par gravité.

2. Un collecteur (20) adapté pour fournir au moins un échantillon de fluide à un analyseur dans un système pour mesurer au moins un analyte dans ledit échantillon de fluide, ledit collecteur (20) comprenant :
- un corps (25),
- dans le corps (25), pour chaque échantillon de fluide à analyser, une cellule fluidique comprenant un canal de distribution de fluide (21) et un canal de collecte (22a, 22b) en communication fluidique avec le canal de distribution de fluide (21) ;
dans lequel un tube de distribution de fluide (30) peut être ajusté avec le canal de distribution de fluide (21), de sorte qu'il émerge dans le canal de collecte (22a, 22b), et dans lequel un préleveur d'échantillon peut être mis en contact avec au moins une goutte (10) de l'échantillon de fluide émergeant au sommet du tube de distribution de fluide (30) en tant que volume de contact et peut transférer une aliquote de l'échantillon de fluide vers un analyseur pour la mesure et/ou l'analyse ;
ou
dans lequel un tube de distribution de fluide (30) peut être ajusté avec le canal de distribution de fluide (21) en connexion fluidique avec un puits de mesure (40), de sorte que le puits de mesure (40) peut être rempli avec l'échantillon de fluide, et dans lequel un préleveur d'échantillon peut être immergé dans le puits de mesure (40) de sorte qu'il peut prélever une aliquote de l'échantillon de fluide et la transférer vers un analyseur pour la mesure et/ou l'analyse ;
- et dans lequel lesdits canaux de collecte (22a, 22b) sont plongeants de sorte que l'échantillon de fluide peut être collecté par gravité.

3. Le collecteur (20) selon la revendication 1 ou 2, comprenant une pluralité de cellules fluidiques.

4. Le collecteur (20) selon l'une quelconque des revendications 1 à 3, dans lequel les canaux de collecte (22a, 22b) sont en connexion fluidique avec un analyseur, ou sont en connexion fluidique avec un dispositif de stockage, par exemple une plaque multipuits.

5. Le collecteur (20) selon l'une quelconque des revendications 1 à 3, dans lequel le corps (25) comprend en outre un canal principal de déchets (26) ou un entonnoir principal de déchets en connexion fluidique avec les canaux de collecte (22a, 22b).

6. Le collecteur (20) selon l'une quelconque des revendications précédentes, comprenant un canal de distribution principal (32) en connexion fluidique avec le tube de distribution de fluide (30) et avec le ou les canaux de distribution de fluide (21).

7. Le collecteur (20) selon l'une quelconque des revendications 2 à 5, dans lequel l'analyseur peut être adapté pour effectuer une analyse choisie dans le groupe comprenant l'analyse d'interaction biomoléculaire, l'interférométrie biophotonique, l'analyse par résonance plasmonique de surface (SPR), la spectroscopie Raman exaltée de surface (SERS), la spectroscopie optique, l'électrophorèse capillaire, la chromatographie liquide (LC), la chromatographie liquide à haute performance (HPLC), l'analyse par diffusion de la lumière à angles multiples (MALS), l'analyse par diffusion dynamique de la lumière (DLS), la spectrométrie de masse, l'imagerie cellulaire, la cytométrie en flux et/ou la réaction en chaîne par polymérase (PCR).

8. Le collecteur (20) selon l'une quelconque des revendications 2 à 6, dans lequel le préleveur d'échantillon peut être une aiguille, une seringue, une pipette automatique ou un robot pipetteur, un tube, un tube flexible, une ligne et/ou un tuyau en connexion fluidique avec l'analyseur.

9. Le collecteur (20) selon l'une quelconque des revendications précédentes, comprenant en outre une jonction liquide, par exemple une vanne trois voies dans le tube de distribution de fluide (30), connectée à un canal de collecte alternatif, de sorte que l'échantillon de fluide peut être retiré du volume de contact, de la goutte (10) ou du puits de mesure (40) à travers le canal de distribution de fluide (21) par une force d'entraînement hydrodynamique, par exemple par une pompe ou une seringue, de préférence
dans lequel l'échantillon de fluide collecté par l'utilisation de la jonction liquide dans le tube de distribution de fluide (30), connecté à un canal de collecte alternatif, peut être dirigé vers un analyseur, ou vers un dispositif de stockage, ou vers un canal de déchets ou un bac à déchets.

10. Un système pour mesurer un ou plusieurs analytes (53) dans des échantillons de fluide discrets provenant d'un récipient adapté pour contenir un fluide, comprenant :
- le collecteur (20) selon l'une quelconque des revendications 1 à 9 ;
- au moins un analyseur ; et
- faculativement un module d'analyse (65) comprenant au moins une ligne d'échantillonnage principale en connexion fluidique avec le récipient et une ou plusieurs pompes en connexion fluidique avec ladite ou lesdites lignes d'échantillonnage principales et adapté pour pomper sélectivement le fluide dans la ligne d'échantillonnage principale en s'éloignant du récipient, comprenant en outre au moins un tube de distribution de fluide (30) en connexion fluidique avec le module d'analyse ;
et/ou
comprenant un moyen pour transférer un échantillon de fluide prélevé manuellement ou automatiquement du récipient dans le système fluidique du module d'analyse (65), ledit moyen étant choisi dans le groupe constitué d'une ou plusieurs seringues, d'une aiguille, d'une pipette manuelle ou automatique, d'un robot pipetteur, d'un tube, d'un tube flexible, d'une ligne et/ou d'un tuyau, de préférence
dans lequel ledit analyseur est tout dispositif adapté pour effectuer une mesure ou une analyse, de préférence dans lequel ladite analyse est une analyse d'interaction biomoléculaire, une interférométrie biophotonique, une analyse par résonance plasmonique de surface (SPR), une spectroscopie Raman exaltée de surface (SERS), une spectroscopie optique, une électrophorèse capillaire, une chromatographie liquide (LC), une chromatographie liquide à haute performance (HPLC), une analyse par diffusion de la lumière à angles multiples (MALS), une spectrométrie de masse, une imagerie cellulaire, une cytométrie en flux et/ou une réaction en chaîne par polymérase (PCR).

11. Un système pour mesurer un ou plusieurs analytes (53) dans des échantillons de fluide discrets provenant d'un récipient adapté pour contenir un fluide, comprenant :
- un ou plusieurs capteurs (5) pour la mesure d'au moins un analyte (53) dans un volume de contact de l'échantillon de fluide ;
- un module d'analyse (65) comprenant au moins une ligne d'échantillonnage principale en connexion fluidique avec le récipient et une ou plusieurs pompes en connexion fluidique avec ladite ou lesdites lignes d'échantillonnage principales et adapté pour pomper sélectivement le fluide dans la ligne d'échantillonnage principale en s'éloignant du récipient ;
- au moins un tube de distribution de fluide (30) en connexion fluidique avec le module d'analyse;
- un collecteur (20) selon l'une quelconque des revendications 1 et 3 à 9 lorsqu'elles dépendent de la revendication 1.

12. Le système selon la revendication 11, dans lequel le capteur est un capteur optique ou un biocapteur.

13. Le système selon l'une quelconque des revendications 11 à 12, dans lequel une pluralité de capteurs est utilisée.

14. Le système selon l'une quelconque des revendications 10 à 13, dans lequel le système comprend au moins un système de bioréacteur multiparallèle, par exemple des récipients de bioréacteur Ambr^{®}, et un système de capteurs d'interférométrie biophotonique, par exemple Octet^{®}.

15. Le système selon l'une quelconque des revendications 10 à 14, comprenant en outre un système de contrôle de flux automatisé adapté pour :
(a) configurer ledit système de contrôle de flux et stocker une configuration de remplissage d'échantillon pour le module d'analyse, la configuration de remplissage d'échantillon comprenant une période prescrite et/ou un volume prescrit pour créer le volume de contact ;
(b) faire fonctionner automatiquement ledit système d'échantillonnage dans une direction d'échantillonnage en s'éloignant du récipient dans le collecteur (20) en utilisant la configuration de remplissage ;
(c) configurer et stocker une configuration de contact pour le module d'analyse, dans laquelle le flux d'échantillon est arrêté pendant une période de contact prescrite, au cours de laquelle une analyse est automatiquement lancée et le capteur (5) est mis en contact avec le volume de contact, et/ou le préleveur d'échantillon est mis en contact avec le volume de contact ;
(d) faire fonctionner automatiquement ledit système d'échantillonnage en utilisant la configuration de contact ; et/ou
(e) configurer et stocker une configuration de nettoyage pour le système, de préférence pour le module d'analyse, le nettoyage étant réalisé en poussant l'échantillon de fluide et/ou le fluide de nettoyage depuis le capteur (5) vers les canaux de collecte (22a, 22b), ladite configuration de nettoyage comprenant une ou plusieurs périodes de nettoyage et/ou un ou plusieurs volumes de nettoyage ;
(f) faire fonctionner automatiquement le système en utilisant la configuration de nettoyage.

16. Une méthode pour mesurer des analytes dans des échantillons de fluide discrets provenant d'un récipient adapté pour contenir un fluide, comprenant les étapes de :
a) pomper l'échantillon de fluide du récipient dans un collecteur (20) selon l'une quelconque des revendications 1 et 3 à 9 pour créer un volume de contact d'échantillon de fluide dans les cellules fluidiques du collecteur (20) ;
ou
transférer l'échantillon de fluide prélevé du récipient dans un collecteur (20) selon l'une quelconque des revendications 2 à 9 en transférant l'échantillon de fluide prélevé manuellement ou automatiquement du récipient dans le système fluidique du collecteur (20), ledit transfert pouvant être effectué en utilisant une ou plusieurs seringues, une aiguille, une pipette manuelle ou automatique, une pipette pour créer un volume de contact d'échantillon de fluide dans les cellules fluidiques du collecteur (20) ;
b) mettre en contact le capteur (5) avec le volume de contact et effectuer une mesure pendant une période de contact ;
ou
mettre en contact un préleveur d'échantillon avec au moins une goutte (10) de l'échantillon de fluide émergeant au sommet du tube de distribution de fluide (30) en tant que volume de contact et transférer une aliquote de l'échantillon de fluide vers un analyseur pour effectuer la mesure et/ou l'analyse ;
ou
immerger un préleveur d'échantillon dans le puits de mesure (40), prélever une aliquote de l'échantillon de fluide avec le préleveur d'échantillon, et transférer une aliquote de l'échantillon de fluide vers un analyseur pour effectuer la mesure et/ou l'analyse ;
c) transférer l'échantillon de fluide dans les canaux de collecte (22a, 22b) et éliminer l'échantillon de fluide par gravité ;
ou
transférer l'échantillon de fluide dans les canaux de collecte (22a, 22b) qui sont en connexion fluidique avec un analyseur pour une mesure complémentaire, ou collecter l'échantillon de fluide par gravité dans un dispositif de stockage, par exemple une plaque multipuits ;
d) transmettre et/ou transférer la mesure sur une interface utilisateur ou vers un dispositif de surveillance de procédé en ligne ou un système de contrôle de procédé.
